# EUROPÄISCHE PATENTANMELDUNG

(11) **EP 2 842 515 A1**
(43) Veröffentlichungstag der Anmeldung: **04.03.2015**
(21) Anmeldenummer: 13182248.8
(22) Anmeldetag: 29.08.2013
(51) Int. Cl.: A61C 8/02, A61L 27/18, A61L 31/06, A61F 2/28, A61L 15/26

(54) **Membran, Operations-Set mit Membran und Verfahren zum Applizieren einer Membran**

(71) Anmelder: nolax AG, 6203 Sempach Station (CH)
(72) Erfinder: Häfner, Stephan, 8057 Zürich (CH); Buser, Stephan, 6210 Sursee (CH)
(74) Vertreter: Hepp Wenger Ryffel AG

(57) **Zusammenfassung**

Die Erfindung betrifft eine Membran (10; 10'), enthaltend mindestens ein Loch (11), welches derart ausgebildet ist, dass die Zahnwurzel (21) mindestens eines Zahnes (20) oder der Implantatkörper mindestens eines Zahnimplantates, bevorzugt die Zahnwurzeln (21) mehrerer benachbarter Zähne (20) oder die Implantatkörper mehrerer benachbarter Zahnimplantate, von dem Loch (11) bzw. den Löchern (11) insbesondere im Wesentlichen manschettenartig umschliessbar ist, wobei die Membran (10; 10') mindestens ein synthetisches und resorbierbares Material enthält oder daraus besteht. Die Membran (10; 10') kann Polyurethan enthalten oder daraus bestehen. Offenbart sind zudem ein Operations-Set enthaltend mindestens eine Membran (10; 10') und ein Verfahren zum Applizieren einer Membran (10; 10').

## Beschreibung

Die Erfindung betrifft Membranen, Operations-Sets mit mindestens einer Membran sowie Verfahren zum Applizieren einer Membran.

Gewebedefekte im Kiefer- und Zahnbereich sind häufig und grösstenteils bedingt durch bakterielle Infektionen mit chronisch entzündlichen Prozessen. Speziell der Zahnhalteapparat (Parodont) ist infolge einer Schwachstelle, dem manschettenartigen Saumepithel um die Zahnwurzel im Bereich der Schmelz-Zementgrenze, betroffen. Dieses Saumepithel bildet die physiologische Abdichtung um einen Zahn und isoliert das darunterliegende Gewebe (Desmodont und Alveolarknochen) von der Mundhöhle. Kommt es zum Beispiel infolge unzureichender Mundhygiene zu chronischen Zahnfleisch-Entzündungen, wird dieses Saumepithel gelockert und bildet die Eintrittspforte für Bakterien ins Parodont. Unbehandelt bilden sich zunehmend tiefere Zahnfleisch- und anschliessend Knochentaschen, verursacht durch entzündliche Resorptionen des Zahnhalteapparates. Bei mehrwurzeligen Zähnen sind Furkationsdefekte die Folge. Progressive Zahnlockerung, Schmerzen und schlussendlich Zahnverlust können resultieren. Solche parodontalen Defekte sind immer Herdinfektionen mit bedeutenden systemischen Konsequenzen und verantwortlich für verschiedene Herderkrankungen. Es besteht folglich das Bestreben, solche Gewebedefekte des Zahnhalteapparates geeignet zu sanieren und zu regenerieren. Durch gründliche mechanische und chemische Reinigung solcher Defekte können wieder entzündungsfreie Verhältnisse geschaffen werden und sekundär die Regeneration des verloren gegangenen Stützgewebes mittels der sogenannten "Guided Tissue Regeneration" (GTR) unterstützt werden.

Die beschriebenen Gewebedefekte und Therapie-Möglichkeiten sind ebenfalls bei periimplantären Läsionen zu finden resp. anwendbar. Bei regulär osseointegrierten Zahnimplantaten besteht eine direkte Implantat-Knochenverbindung ohne desmodontale Zwischenstruktur. Das Zahnimplantat ist somit direkt knöchern im Kieferknochen verankert, analog einer Zahn-Ankylose. Durch chronisch entzündliche Prozesse kommt es entlang der Implantat-Oberfläche zum Knochenabbau. Durch die Freilegung der porösen Oberfläche und der Gewindeanteile von cervical nach apikal des Zahnimplantates erfolgt eine zunehmende Lockerung, was letztendlich zu dessen Verlust führt.

Die GTR ist eine etablierte Methode in der Zahnmedizin und speziell in der Parodontologie, um Gewebe-Defizite im Bereich des Zahnhalteapparates zu regenerieren. Die Herausforderung besteht dabei im Wettlauf zwischen langsam wachsendem Hartgewebe (Knochen und Desmodont) und schnell wachsendem Weichgewebe (Bindegewebe, Mukosa). Diese unterschiedlich schnell wachsenden Gewebe müssen mit einer geeigneten Struktur voneinander getrennt werden, um die parodontale Regeneration vor Weichgewebe-Invasion zu schützen und damit den zu regenerierenden Geweben genügend Volumen zur Verfügung zu stellen. Es ist bekannt, dazu sowohl resorbierbare als auch nicht-resorbierbare Membranen zu verwenden. Derartige Membranen dienen der zelldichten Gewebeseparation und verhindern das Einwachsen von Weichgewebe (z.B. Saumepithel) entlang der Hartgewebe-Defektoberflächen. Gleichzeitig funktionieren sie als gewebeverträgliche Leitstruktur mit einer begrenzten Eigenstabilität. Sie stellen somit das zentrale Instrument der GTR dar. Membranen aus verschiedenen Materialien stehen heute zur Verfügung, keine jedoch vermag die massgebenden Anforderungen zu kombinieren.

Bekannte nicht-resorbierbare Membranen bestehen hauptsächlich aus mikroporösem Teflon (ePTFE) oder Titan-Gittern. Spezielle nicht-resorbierbare Membranen sind beispielsweise in der DE 94 16 010 U1 und der EP 0 621 013 A2 offenbart. Ein Vorteil solcher Membranen liegt in der Formbarkeit und in der hohen Formstabilität. Sie können individuell modelliert und an die Defektverhältnisse angepasst werden und sichern dadurch langzeitstabil das gewünschte Volumen zur Geweberegeneration. Sie haben jedoch entscheidende Nachteile. Einerseits müssen sie zwingend fixiert werden, was über zusätzliche Schrauben, Pins oder Nähte erreicht wird. Andererseits führt ihre rigide Struktur oft zu Weichgewebs-Dehiszenzen mit erhöhtem Infektionsrisiko bei ohnehin bereits erhöhter Infektionsanfälligkeit. Der Hauptnachteil liegt jedoch klar in der Notwendigkeit eines sekundären chirurgischen Eingriffs zur Entfernung der Membran und allfälliger Fixierungsmittel. Dies bedeutet zusätzliche Belastung für den Patienten aus medizinischer, psychischer und finanzieller Sicht. Darüber hinaus wird erneutes Narbengewebe provoziert.

Resorbierbare Membranen müssen im Idealfall nicht mehr sekundär chirurgisch entfernt werden und ermöglichen somit einzeitige GTR-Eingriffe. Hauptsächlich werden dazu heute Kollagen-Membranen verwendet, wie beispielsweise Bio-Gide® von der Geistlich Pharma AG (siehe EP 1 676 592 A1), welches porcines Kollagen enthält, oder TissuFoil E von Resorba, welches equines Kollagen enthält. Sie sind tierischer Herkunft und besitzen potentielle Infektionsrisiken (z.B. Prionen) sowie allgemein erhöhte Allergierisiken. Patientenseitig reduzieren diese Nachteile die Akzeptanz gegenüber solchen Materialien. Zudem ist die Herstellung von Membranen aus natürlichen Materialien oftmals aufwändig und vor allem kostenintensiv.

Ein weiterer entscheidender Nachteil dieser Materialien sind aber deren mechanische Eigenschaften speziell im nassen Zustand. Feucht vermögen solche Membranen äusserst beschränkt Zug- und Scherkräfte aufzunehmen und reissen schnell. Zusätzlich kollabieren und gelieren sie durch Feuchtigkeitsaufnahme. Gewebedefekte speziell im Bereich einer Zahnwurzel können somit nicht ausreichend dicht und formstabil gedeckt werden. Zusätzliche Mittel zur Fixierung (resorbierbare Pins oder Nähte) sind notwendig, aber chirurgisch anspruchsvoll und zeitintensiv und ermöglichen dabei dennoch nur beschränkte Resultate speziell im Bereich um eine Zahnwurzel.

Da Kollagen-Biopolymere aus tierischem Gewebe isoliert werden, bestehen nur sehr beschränkt Optimierungsmöglichkeiten in chemischer Hinsicht. Dies widerspiegelt sich in der Verlängerung der optimalen Resorptionszeit, welche tendenziell zu kurz sind. Zudem sind sie auch über Quervernetzungen nicht zufriedenstellend modifizierbar.

Behandlerseitige Abhilfe durch Anwendung doppelter Schichten ist unbefriedigend, erhöht die Volumenforderung und speziell die Kosten. Kollagen-Membranen sind dabei infolge der aufwendigen Herstellung bereits sehr teuer und kommen somit bei weitem nicht für alle Patienten mit entsprechendem Behandlungsbedarf in Frage.

Weitere resorbierbare Membranen sind beispielsweise in der WO 97/32616 A1 oder der WO 90/13302 A1 offenbart.

Synthetische resorbierbare Membranen sind ebenfalls erhältlich, wie beispielsweise die PLLA-Membranen BioCellect von Imtec Corporation oder die PGA-Membranen Vicryl von Ethicon Products. PLLA und PGA besitzen jedoch infolge Gewebeansäuerung während der Resorption deutlich nachteilige Effekte auf die Wundheilung. Membragel®, ein PEG-Hydrogel von Straumann, wird in flüssiger Form aufgetragen und härtet innerhalb von 60 Sekunden durch Feuchtigkeitsaufnahme zu einer "Membran" aus. Durch die anspruchsvolle Handhabung und die bisherigen dürftigen klinischen Resultate konnte sich dieses Konzept jedoch nicht durchsetzen. Die vom Hersteller vorgeschriebene individuelle Anwenderschulung verdeutlicht die Problematik.

Eine ideale GTR-Membran für allgemeine, aber vor allem auch spezielle parodontologische Regenerationen, existiert soweit nicht.

Es ist daher eine Aufgabe der vorliegenden Erfindung, eine Membran bereitzustellen, mit der die oben beschriebenen Nachteile des Standes der Technik zumindest teilweise behoben oder sogar gänzlich ausgeräumt werden können. Insbesondere sollte eine solche Membran robust sein, auch im nassen Zustand nicht verkleben, aber dennoch ausreichend auf dem Gewebegrund haften, und einfach zu applizieren sein und dabei dieselben mechanischen Eigenschaften (Eigensteifigkeit, Flexibilität, Elastizität, Reissfestigkeit) im trockenen und nassen Zustand aufweisen, so dass sie ohne oder mit einer Vorbehandlung (insbesondere Anfeuchtung) anwendbar ist. Ebenfalls insbesondere sollte eine solche Membran keiner zusätzlichen Fixierung bedürfen. Eine derartige Membran ist insbesondere für die Anwendung an Mensch und Tier als GTR-Implantat vorgesehen, vorzugsweise in der oralen Region.

Diese Aufgabe wird zum einen gelöst durch eine Membran, die mindestens ein Loch enthält, welches derart ausgebildet ist, dass die Zahnwurzel mindestens eines Zahnes oder der Implantatkörper mindestens eines Zahnimplantates, bevorzugt die Zahnwurzeln mehrerer benachbarter Zähne oder die Implantatkörper mehrerer benachbarter Zahnimplantate, von dem Loch bzw. den Löchern umschliessbar ist. Erfindungsgemäss enthält die Membran mindestens ein synthetisches und resorbierbares Material.

Unter einer Membran wird hier und im Folgenden eine dreidimensionale, flächige Materialschicht verstanden, mit der zwei Raumbereiche voneinander trennbar sind. Ein erster der beiden Raumbereiche kann beispielsweise für das Wachsen von Hartgewebe vorgesehen sein. Bei dem ersten Raumbereich kann es sich etwa um eine Knochentasche handeln. Ein zweiter der beiden Raumbereiche kann für das Wachsen von Weichgewebe vorgesehen sein kann. Vorteilhafterweise ist die Membran gasdurchlässig, insbesondere wasserdampfdurchlässig. Hierdurch kann die Regeneration des menschlichen oder tierischen Gewebes unterstützt werden. Die Wasserdampfdurchlässigkeit wird im Rahmen der Erfindung gemäss Abschnitt 3.2 der Norm DIN EN 13726-2:2002 bestimmt, wo sie auch als Feuchtigkeitstransportrate bei Kontakt mit Wasserdampf bezeichnet wird. Bevorzugt ist die Wasserdampfdurchlässigkeit mindestens 100 g/(m²·24 h), bevorzugt mindestens 200 g/(m²·24 h) und besonders bevorzugt mindestens 300 g/(m²·24 h) - insbesondere wenn als Prüfdauer gemäss Abschnitt 3.2.3.6 der genannten Norm eine Zeit von 24 h gewählt wird. In vielen Ausführungsformen ist die Wasserdampfdurchlässigkeit höchstens 400000 g/(m²·24 h), oder höchstens 200000 g/(m²·24 h), oder höchstens 50000 g/(m²·24 h) - insbesondere wenn als Prüfdauer gemäss Abschnitt 3.2.3.6 der genannten Norm eine Zeit von 24 h gewählt wird. Für die Durchführung des Prüfverfahrens wird auf die Beschreibung in den Ausführungsbeispielen verwiesen.

Bevorzugt ist die Membran ebenfalls wasserdurchlässig. Hierdurch kann die Regeneration des menschlichen oder tierischen Gewebes noch weiter unterstützt werden. Die Wasserdurchlässigkeit wird im Rahmen der Erfindung gemäss Abschnitt 3.3 der Norm DIN EN 13726-2:2002 bestimmt, wo sie auch als Feuchtigkeitstransportrate bei Kontakt mit Flüssigkeit bezeichnet wird. Vorteilhafterweise verfügt die Membran über eine Wasserdurchlässigkeit von mindestens 100 g/(m²·24 h), bevorzugt mindestens 300 g/(m²·24 h) und besonders bevorzugt mindestens 500 g/(m²·24 h) - insbesondere wenn als Prüfdauer gemäss Abschnitt 3.3.3.3 der genannten Norm eine Zeit von 4 h gewählt wird. In vielen Ausführungsformen ist die Wasserdurchlässigkeit höchstens 400000 g/(m²·24 h), oder höchstens 200000 g/(m²·24 h), oder höchstens 50000 g/(m²·24 h) - insbesondere wenn als Prüfdauer gemäss Abschnitt 3.3.3.3 der genannten Norm eine Zeit von 4 h gewählt wird.

Ebenfalls von Vorteil ist es, wenn die Membran zelldicht ist - wenn also der Durchtritt von menschlichen oder tierischen Zellen durch die Membran hindurch verhindert ist. Aufgrund der Zelldichtheit der Membran kann das Einwachsen von Weichgewebe aus dem zweiten Raumbereich in den ersten Raumbereich verhindert werden. Zu diesem Zweck ist es bevorzugt, wenn die Membran keine Poren mit Porendurchmessern von mehr als 5 µm enthält.

Von weiterem Vorteil ist es, wenn die Membran ausserdem undurchlässig ist für Mikroorganismen wie bspw. Bakterien (insbesondere Mikroorganismen bzw. Bakterien mit Durchmessern im Bereich von 0,5 µm bis 10 µm) und/oder Viren (insbesondere mit Durchmessern im Bereich von 0,01 µm bis 0,1 µm). Dies kann ebenfalls die Regeneration des menschlichen oder tierischen Gewebes positiv beeinflussen. Hierfür ist es bevorzugt, wenn die Membran keine Poren mit Porendurchmessern von mehr als 0,5 µm, weiter bevorzugt keine Poren mit Porendurchmessern von mehr als 0,1 µm und besonders bevorzugt keine Poren mit Porendurchmessern von mehr als 0,01 µm aufweist.

Im Rahmen der Erfindung wird ein Material als synthetisch angesehen, wenn es nur künstlich herstellbar ist, also nicht natürlichen Ursprungs ist oder strukturell mit einem Material natürlichen Ursprungs übereinstimmt. Insbesondere schliesst der Begriff "synthetisch" also Materialien tierischer Herkunft, wie beispielsweise Kollagen, aus.

Als resorbierbar wird ein Material im Rahmen der Erfindung bezeichnet, wenn es vom menschlichen oder tierischen Körper zumindest teilweise oder sogar vollständig zersetzbar ist, insbesondere durch Hydrolyse, Oxidation und/oder enzymatische Spaltung.

Die erfindungsgemässe Membran kann in einigen Ausführungsformen auch Anteile nicht-synthetischer und/oder nicht-resorbierbarer Materialien enthalten, beispielsweise Biopolymere wie Kollagene, Glykosaminoglykane, Cellulose, Chitin, Chitosan oder beliebige Kombinationen davon. Es ist jedoch bevorzugt, wenn mindestens 90 Gew.-%, bevorzugt mindestens 95 Gew.-% und weiter bevorzugt mindestens 99 Gew.-% synthetische und resorbierbare Materialien enthält. Besonders bevorzugt besteht die Membran vollständig aus einem oder mehreren synthetischen und resorbierbaren Materialien. Besteht die Membran nicht vollständig aus einem oder mehreren synthetischen und resorbierbaren Materialien, so bestehen die verbleibenden Inhaltsstoffen bevorzugt aus den im folgenden noch detailliert beschriebenen Bioziden, Wachstumsfaktoren und beliebigen Kombinationen davon.

Besonders bevorzugt ist das mindestens eine Loch der Membran derart ausgebildet, dass die Zahnwurzel oder Zahnwurzeln, der Zahn-Implantatkörper oder die Zahn-Implantatkörper damit im Wesentlichen manschettenartig umschliessbar ist bzw. sind. Insbesondere kann der Rand des Lochs im implantierten Zustand im Wesentlichen vollständig in Kontakt mit der Zahnwurzel oder dem Zahn-Implantatkörper sein. Dabei bedeutet "im Wesentlichen vollständig in Kontakt", dass sich der Rand des Lochs zumindest an die grobe Oberflächenkontur der Zahnwurzel unter Spannung anschmiegt. Auf der Grössenskala der natürlichen Oberflächenrauigkeit der Zahnwurzel kann der Rand des Lochs jedoch einen geringfügigen Abstand von der Zahnwurzel aufweisen. Die Membran kann somit vorübergehend die Funktion des Saumepithels ersetzen und sicher die Invasion von Weichgewebe in den zu regenerierenden Defekt verhindern.

Um die Zahnwurzel eines einzelnen Zahnes im Wesentlichen manschettenartig umschliessen zu können, kann das Loch der Membran im kräftefreien Zustand einen Durchmesser haben, der bevorzugt kleiner ist als der Durchmesser der Zahnwurzel. So kann etwa der Durchmesser des Lochs im Bereich von 0,1 mm bis 20 mm liegen, bevorzugt von 0,4 mm bis 12 mm, besonders bevorzugt von 0,5 mm bis 8 mm. Unter dem Durchmesser eines Lochs wird der Durchmesser des Hüllkreises dieses Lochs verstanden, also der kleinste Durchmesser eines Kreises, welcher das Loch umschliesst. Das Loch kann kreisförmig sein oder auch eine andere Form haben, die ein Umschliessen einer Zahlwurzel ermöglicht. Um die Zahnwurzeln mehrerer benachbarter Zähne umschliessen zu können, kann die Membran mehrere Löcher enthalten. Diese Löcher können identische oder verschiedene Durchmesser aufweisen, die jeweils in den oben genannten Bereichen liegen können. Die Löcher können entlang einer individuellen Zahnbogenkontur mit individuellen Abständen in die Membran eingearbeitet sein oder eingearbeitet werden.

Die erfindungsgemässen Membranen zeichnen sich durch eine Reihe von Vorteilen aus. So sind sie beispielsweise robust, sie verkleben kaum oder gar nicht (weder im trockenen noch im nassen Zustand) und sind daher einfach zu applizieren. Zudem haben sie im trockenen und im nassen Zustand zumindest annähernd dieselben mechanischen Eigenschaften (wie etwa Eigensteifigkeit, Flexibilität und speziell Elastizität und/oder Reissfestigkeit). Sie können daher je nach medizinischer Indikation im trockenen und nassen Zustand angewendet werden, was die chirurgische Handhabung erleichtert. Aufgrund ihrer Elastizität und Reissfestigkeit bedürfen die Membranen nicht zwingend einer zusätzlichen Fixierung, obgleich eine zusätzliche Fixierung bei einigen medizinischen Indikationen vorteilhaft sein kann. Insbesondere bei der weiter unten noch im Detail beschriebenen subgingivalen Verwendung kann die Membran vorübergehend die Funktion des Saumepithels übernehmen und eine Abdichtung zwischen einem ersten Raumbereich, in dem sich Hartgewebe gebildet, und einem zweiten Raumbereich, in dem sich Weichgewebe bildet, ermöglichen. Gegenüber den aus dem Stand der Technik bekannten Membranen aus Kollagen verfügen die erfindungsgemässen Membranen über eine deutlich erhöhte Volumenstabilität, da im nassen Zustand weder ein Kollabieren noch ein Gelieren auftreten, je nach Ausführungsform aber ein minimes oder deutliches Quellen erreicht werden kann. Zudem werden das bei Membranen aus tierischem Material bestehende Allergiepotential und Kontaminationsrisiko deutlich reduziert.

Die Membran kann Polyurethan enthalten oder daraus bestehen. Die Verwendung von Polyurethan ist in der Medizin und insbesondere der Wundheilung seit den 1960er Jahren bekannt. Aromatische Polyurethane zeichnen sich durch ihre Biokompatibilität und Biostabilität aus. In neuerer Zeit sind vermehrt aliphatische Polyurethane von Interesse, mit welchen sich resorbierbare oder bioabbaubare Materialien realisieren lassen, deren Degradationsprodukte biologisch und physiologisch unbedenklich sind, also weder toxisch noch mutagen sind.

Bevorzugte synthetische resorbierbare Polyurethan-Materialien an sich sind beispielsweise in der WO 2012/062700 beschrieben. Die in der WO 2012/062700, insbesondere in deren Patentansprüchen 1 bis 8 offenbarten chemischen Zusammensetzungen des Polyurethan-Materials werden durch Bezugnahme in die vorliegende Anmeldung aufgenommen. Auch bezüglich der Vorteile von Membranen dieser Zusammensetzungen wird auf die WO 2012/062700 verwiesen. Weitere bevorzugte synthetische resorbierbare Polyurethan-Materialien an sich sind in der WO 2011/015568 beschrieben, die sich jedoch mit Wundabdeckungen aus Polyurethan-Schäumen befasst, nicht aber mit Membranen. Auch auf die WO 2011/015568, insbesondere in deren Patentansprüchen 1 bis 11 offenbarten chemischen Zusammensetzungen des Polyurethan-Materials werden durch Bezugnahme in die vorliegende Anmeldung aufgenommen.

Insbesondere kann die Membran aus einer Zusammensetzung hergestellt oder herstellbar sein, welche enthält:
- wenigstens eine Polyol-Komponente enthaltend wenigstens einen Stoff mit wenigstens zwei, bevorzugt zwei, drei oder vier Hydroxyl-Gruppen;
- wenigstens eine Polyisocyanat-Komponente oder wenigstens ein Polyurethan-Präpolymer enthaltend wenigstens einen Stoff mit wenigstens zwei, bevorzugt zwei, drei oder vier Isocyanat-Gruppen;
- ein Additiv enthaltend wenigstens einen der folgenden Stoffe: Polyvinylpyrrolidon, Polyvinylpolypyrrolidon, ein Copolymer aus Polyvinylpyrrolidon mit Vinylacetat, Vinylcaprolactam oder Vinylimidazol, oder Mischungen davon.

Die Membran kann mindestens eines der folgenden Polymere enthalten oder daraus bestehen: Poly-α-Hydroxyester, Polyorthoester, Polyetherester, Polyanhydrid, Polyphosphazen, Polypropylenfumarat, Polyesteramid, Polyethylenfumarat, Polyaminosäuren, Polysaccharide, Polypeptide, Polyhydroxybutyrat, Polyhydroxyvalerates, Polymalat, Poly(Milchsäure), Poly(Glykolsäure), Polycaprolacton, Poly(glycolid-co-trimethylencarbonat), Polydioxanon, Polyethylenglycol, sowie Gemische aus diesen Polymeren. Zusätzlich kann die Membran mindestens einen der folgenden anorganischen Bestandteile enthalten: Calciumsulfat, Calciumphosphat, wie zum Beispiel Mono-Calciumphosphat-monohydrat, Mono-Calciumphosphat wasserfrei, Di-Calciumphosphat dihydrat, Di-Calciumphosphat wasserfrei, Tetracalciumphosphat, Calciumorthophosphat, Calciumpyrophosphat, α-Tricalciumphosphat, β-Tricalciumphosphat, Apatit, sowie Hydroxyapatit. Derartige Bestandteile können die mechanischen Eigenschaften der Membran beeinflussen (insbesondere kann eine Versteifung bewirkt werden), oder es kann der Abbau zeitlich beeinflusst bzw. die Bildung von Hart- und/oder Weichgewebe stimuliert werden.

Die Zusammensetzung, aus der die erfindungsgemässe Membran herstellbar ist, kann weiterhin optional mindestens eine der folgenden Komponenten enthalten:
- mindestens einen Katalysator, insbesondere mindestens einen Urethan-Katalysator und/oder mindestens einen Aminkatalysator, wie beispielsweise 2,2-Dimorpholinodiethylether (DMDEE);
- mindestens einen Stabilisator, wie beispielsweise Tocopherol;
- mindestens einen Porenbildner, wie beispielsweise Calciumstearat;
- mindestens einen Emulgator, wie beispielsweise Sympatens TRH/400;

Die erfindungsgemässen Membranen können durch an sich bekannte Verfahren hergestellt und anschliessend mit einem oder mehreren Löchern versehen werden. Bevorzugt wird die erfindungsgemässe Membran hergestellt durch Sprühauftrag oder Ausrakeln eines Polymers auf einem Substrat, Aushärten des Polymers und anschliessendes Entfernen des Substrats. Das Substrat kann beispielsweise silikonisiertes Papier oder eine Stahloberfläche sein. Auf diese Weise lassen sich besonders einfach Membranen mit mehreren Schichten erreichen, so wie sie weiter unten noch detailliert beschrieben werden. Ebenfalls möglich ist eine Walzenauftragung, bei der das Polymer mit Hilfe mindestens einer Walze auf das Substrat aufgetragen wird. Alternativ kann die erfindungsgemässe Membran auch durch eine Extrusion hergestellt werden, bei der ein ausreagiertes Polymer zu einem Granulat verarbeitet, dieses erhitzt und über hohen Druck durch eine Breitschlitzdüse auf ein Förderband extrudiert wird. Des Weiteren kann die erfindungsgemässe Membran auch durch eine Vorhangbeschichtung ("curtain coating") des noch nicht ausreagierten Polymers hergestellt werden; dieses Verfahren ist besonders für die Herstellung mehrschichtiger Membranen geeignet.

Die Membran kann eine Dicke aufweisen, die im Bereich von 0,01 mm bis 4 mm, bevorzugt von 0,05 mm bis 1,0 mm, besonders bevorzugt von 0,1 mm bis 0,8 mm liegt. Die Dicke richtet sich nach dem Therapieziel und kann vom Fachmann anhand dieses Therapieziels ausgewählt werden. Unter der Dicke wird hier die Gesamtdicke der Membran verstanden, die (wie weiter unten noch im Detail beschrieben wird) eine oder auch mehrere Schichten enthalten kann.

Bevorzugt enthält die Membran Poren, sie kann aber auch porenfrei ausgebildet sein. Diese Poren können Porendurchmesser im Bereich von 0,0001 mm bis 0,05 mm, bevorzugt von 0,0005 mm bis 0,01 mm, besonders bevorzugt von 0,001 mm bis 0,005 mm aufweisen. Die Poren beeinflussen die Gasdurchlässigkeit (insbesondere die Wasserdampfdurchlässigkeit), die Wasserdurchlässigkeit der Membran und ihre Undurchlässigkeit für Mikroorganismen (insbesondere mit Grössen im Bereich von 0,6 µm bis 1 µm oder sogar Grössen im Bereich von 0,01 µm bis 10 µm).

Ebenfalls von Vorteil ist es, wenn die Membran transparent ist. Dies erleichtert die Positionierung der Membran und erlaubt eine Kontrolle von Gewebe, welche sich unterhalb der Membran befindet.

Durch Wahl der chemischen Zusammensetzung, der Dicke und der Porendurchmesser kann der Fachmann die Eigenschaften der Membran einstellen. Beispielsweise lässt sich hierdurch der Zeitraum einstellen, innerhalb dessen die Membran vom menschlichen oder tierischen Körper vollständig resorbiert wird. Dieser Zeitraum kann sich im Bereich von wenigen Tagen bis zu mehreren Monaten erstrecken. Zudem kann durch die Wahl der chemischen Zusammensetzung, der Dicke und der Porendurchmesser eine Zelldichtigkeit, eine Gasdurchlässigkeit, eine Wasserdurchlässigkeit und eine Transparenz eingestellt werden.

Die Membran kann mindestens ein Biozid enthalten. Unter einem Biozid wird ein Wirkstoff verstanden, der auf chemischem oder biologischem Wege Schadorganismen zerstören, abschrecken, unschädlich machen, Schädigungen durch sie verhindern oder sie in anderer Weise bekämpfen kann, der jedoch dem behandelten menschlichen oder tierischen Körper keinen Schaden zufügt. Zu den Bioziden gehören beispielsweise Bakterizide, Fungizide, Viruzide, Antibiotika und Antimykotika. Im Rahmen der Erfindung geeignete Biozide sind beispielsweise Polyhexanid, Chlorhexidin, Silber oder Taurolidin, Octenidin-Dihydrochlorid, Cetylpyridiniumchloric, Triclosan oder beliebige Kombinationen davon.

Alternativ oder zusätzlich kann die Membran mindestens einen Wachstumsfaktor enthalten. Darunter wird ein Protein verstanden, das als Signale von einer Zelle auf eine zweite übertragen werden und damit Informationen weiterleiten kann. Im Rahmen der Erfindung geeignete Wachstumsfaktoren sind beispielsweise FGF, TGF, PDGF, EGF, VEG, IGF, NGF und Interleukine.

Das mindestens eine Biozid und/oder der mindestens eine Wachstumsfaktor können im Bereich mindestens einer Oberfläche der Membran vorhanden sein. Dies kann beispielsweise durch eine an sich bekannte Beschichtung der Membran mit dem mindestens einen Biozid und/oder dem mindestens einen Wachstumsfaktor erfolgen.

Alternativ oder zusätzlich können das mindestens eine Biozid und/oder der mindestens eine Wachstumsfaktor im Inneren der Membran vorhanden sein. Hierfür können bereits den Ausgangsstoffen der Membran das mindestens eine Biozid und/oder der mindestens eine Wachstumsfaktor hinzugefügt werden. Beispielsweise können die Ausgangsstoffe zusammen mit dem mindestens einen Biozid und/oder dem mindestens einen Wachstumsfaktor gemeinsam zu einer Membran verarbeitet werden, insbesondere extrudiert oder auf eine Substrat aufgesprüht oder ausgerakelt werden.

Bevorzugt ist die Membran einschichtig ausgebildet, wobei diese eine Schicht mindestens ein synthetisches und resorbierbares Material enthält oder daraus besteht. Es liegt jedoch im Rahmen der Erfindung, dass die Membran mehrere Schichten umfasst, wobei dann jede der Schichten mindestens ein synthetisches und resorbierbares Material enthält oder daraus besteht. Dabei können die Schichten identische oder verschiedene Porositäten aufweisen. Alternativ oder zusätzlichen können die Schichten identische oder verschiedene Dicken aufweisen. Die Dicken der einzelnen Schichten können unabhängig voneinander im Bereich von 0,01 mm bis 3,0 mm liegen.

In einer bevorzugten Variante enthält die Membran eine erste Schicht mit einer Dicke im Bereich von 0,01 mm bis 1,5 mm und einer Porengrösse (Mittelwert aus kleinstem und grösstem Durchmesser der einzelnen Poren, wobei dieser Mittelwert wiederum über alle Poren eines gegebenen Testkörpers gemittelt wird) von maximal 5 µm bei einer Porosität (prozentualer Volumenanteil der Poren am betrachteten Material-Gesamtvolumen) zwischen 0 und 50%; sowie eine zweite Schicht mit einer Dicke im Bereich von 0,01 mm bis 3 mm und einer Porengrösse (wie vorstehend definiert) im Bereich von 0,01 bis 0,6 mm bei einer Porosität (wie vorstehend definiert) im Bereich von 50 bis 90%. Besonders bevorzugt besteht die Membran nur aus diesen beiden Schichten.

Die mehreren Schichten der Membran können deckungsgleich sein. Es ist jedoch denkbar und für einige Verwendungen sogar vorteilhaft, wenn die Schichten nicht deckungsgleich sind. Beispielsweise kann die oben genannte zweite Schicht die oben genannte erste Schicht nur in einem Deckungsbereich bedecken, der nur einen Teilbereich der ersten Schicht bildet. Vorteilhafterweise ist das mindestens eine Loch der Membran ausserhalb des Deckungsbereichs angeordnet; es durchdringt also nur die erste Schicht, nicht aber auch die zweite Schicht. Dies ermöglicht es, dass nur die erste Schicht die Zahnwurzel oder den Implantatkörper manschettenartig umschliessen kann. Die Membran kann so platziert werden, dass die zweite Schicht in Richtung des Weichgewebes zu liegen kommt, ohne dass diese jedoch einen Kontakt zur Zahnwurzel oder zum Implantatkörper hat. Diese Ausführungsform hat den Vorteil, dass die Membran im Bereich der Zahnwurzel oder des Implantatkörpers eine geringere Dicke aufweist, wodurch sie sich hier besser an die Zahnwurzel bzw. den Implantatkörper anschmiegen kann. Die zweite Schicht, die nur in einem von der Zahnwurzel bzw. dem Implantatkörper beabstandeten Bereich vorliegt, kann einen zusätzlichen Heilungseffekt bewirken, indem sie das Wachstum von Weichgewebe fördert. Die zweite Schicht kann aber auch gegen den Knochen platziert werden.

Wie oben beschriebene Membranen eignen sich speziell für die Regeneration parodontaler Defekte (insbesondere ein- bis mehrwandige Knochentaschen und Furkationsdefekte), da sie wie ein "subgingivaler Kofferdam" appliziert werden können. Ein solches Verfahren kann die folgenden Schritte enthalten:
a) Bereitstellen einer wie oben beschriebenen Membran mit mindestens einem Loch oder Einbringen mindestens eines Lochs in eine wie oben beschriebene Membran;
b) Applizieren der Membran auf einen Kieferknochen derart, dass das Loch die Zahnwurzel eines Zahnes oder die Zahnwurzeln mehrerer benachbarter Zähne oder eines Zahn-Implantatkörpers oder mehrerer benachbarter Zahn-Implantatkörper insbesondere im Wesentlichen manschettenartig umschliesst.

Des Weiteren eignet sich eine wie oben beschriebene Membran für den zelldichten Verschluss einer Extraktionsalveole zur Mundhöhle, in dem beispielsweise eine solche Membran im Übermass über die leere Alveole gespannt und subgingival um die angrenzenden Zahnwurzeln fixiert wird.

In beiden Verfahren ermöglichen die Elastizität und Flexibilität der Membran den anhaltend dichten und vertikal stabilen Defekt-Verschluss im kritischen cervicalen Bereich zirkulär um die Zahnwurzel des Zahnes oder der benachbarten Zähne oder Zahnimplantate. Direkt unterhalb des Zahnfleisches kann die Membran somit temporär die Funktion des Saumepithels ersetzen und gleichzeitig und ohne weitere Fixierung der Separation von Weichgewebe und Defektvolumen dienen. Eine stabile Abdichtung um die Zahnwurzeln oder den Zahn-Implantatkörper und über Ränder des behandelten Defekts hinaus erhöht die Erfolgschancen im Vergleich zu den bekannten Lösungen deutlich. Ein Zweiteingriff erübrigt sich bei komplikationsloser Heilung infolge der Resorption.

Das Einbringen mindestens eines Lochs im Schritt a) kann beispielsweise mit Hilfe einer an sich bekannten Kofferdam-Lochzange erfolgen. Falls erforderlich kann der Membran beispielsweise mit Hilfe einer Schere eine geeignete Grösse und/oder Aussenkontur verliehen werden.

Vor Schritt b) kann das Zahnfleisch auf bekannte Weise aufgeklappt werden, so dass der Kieferknochen freigelegt wird. Die Applikation in Schritt b) kann erleichtert werden, wenn die Membran transparent ist. Nach Schritt b) kann das Zahnfleisch dann wieder auf bekannte Weise reponiert und optional vernäht werden.

Ein weiterer Aspekt der Erfindung, mit dem das oben beschriebene Verfahren durchgeführt werden kann, betrifft ein Operations-Set. Dieses Operations-Set enthält mindestens eine Membran, welche mindestens ein synthetisches und resorbierbares Material enthält oder daraus besteht, sowie
- mindestens ein Lochwerkzeug zum Einbringen mindestens eines Lochs in die Membran, insbesondere mindestens eine Kofferdam-Lochzange, und/oder
- mindestens eine Spannzange zum Spannen der Membran und/oder
- mindestens ein Spreizwerkzeug zur kurzzeitigen Erweiterung eines Interdentalraumes zum erleichterten Applizieren der Membran und/oder
- mindestens eine Schablone zur Vorgabe der relativen Positionen mehrerer in die Membran einzubringender Löcher und/oder
- mindestens einen Stempel zum Markieren der Position mindestens eines in die Membran einzubringenden Lochs und/oder
- mindestens eine Benutzungsanleitung zum Einbringen mindestens eines Lochs in die Membran, insbesondere mit Hilfe mindestens eines Lochwerkzeugs, insbesondere einer Kofferdam-Lochzange.

Bei der Membran des Operations-Sets kann es sich insbesondere um eine wie oben beschriebene Membran mit mindestens einem Loch handeln. Die Membran des Operations-Sets muss jedoch nicht zwingend eines oder mehrere Löcher enthalten, welches derart ausgebildet ist, dass die Zahnwurzel mindestens eines Zahnes oder der Implantatkörper mindestens eines Zahnimplantates, bevorzugt die Zahnwurzeln mehrerer benachbarter Zähne oder die Implantatkörper mehrerer benachbarter Zahnimplantate, von dem Loch bzw. den Löchern insbesondere im Wesentlichen manschettenartig umschliessbar ist. Insbesondere kann die Membran des Operations-Sets auch frei von jeglichen Löchern sein.

Auch eine im Operations-Set enthaltene Membran ohne Löcher kann eine oder mehrere der oben beschriebenen Eigenschaften aufweisen. Beispielsweise kann sie die oben beschriebenen Materialien enthalten oder daraus bestehen; und/oder sie kann eine Dicke in den oben genannten Bereichen aufweisen; und/oder sie kann Poren mit Porendurchmessern in den oben genannten Bereichen aufweisen; und/oder sie kann gasdurchlässig, insbesondere wasserdampfdurchlässig sein und insbesondere eine Wasserdampfdurchlässigkeit in den oben genannten Bereichen aufweisen; und/oder sie kann wasserdurchlässig sein und insbesondere eine Wasserdurchlässigkeit in den oben genannten Bereichen aufweisen; und/oder sie kann transparent sein; und/oder sie kann mindestens ein Biozid und/oder mindestens einen Wachstumsfaktor enthalten, insbesondere mindestens eines/n der oben genannten.

Eine oben genannte Schablone des Operations-Sets kann Markierungen, beispielsweise Aufdrucke enthalten, die die Positionen mehrerer in die Membran einzubringender Löcher vorgeben. Von diesen Löchern können mehrere benachbarte Zahnwurzeln benachbarter Zähne oder mehrere benachbarte Implantatkörper benachbarter Zahnimplantate umschlossen werden. Zum Einbringen der Löcher kann die Schablone flächig mit einer Membran des Operations-Sets in Deckung gebracht werden, zu welchem Zweck sie bevorzugt die gleiche Form und Grösse haben kann wie die Membran. Anschliessend können an den Markierungen Löcher sowohl durch die Membran als auch durch die Schablone hindurch eingebracht werden. Zu diesem Zweck kann ein Lochwerkzeug verwendet werden, insbesondere eine Kofferdam-Lochzange. Dieses Lochwerkzeug kann ebenfalls Bestandteil des Operations-Sets sein. Alternativ kann jedoch auch ein Lochwerkzeug verwendet werden, welches nicht Bestandteil des Operations-Sets ist, sondern beispielsweise in einer Zahnarztpraxis ohnehin vorhanden ist.

Mit einem oben genannten Stempel können eine oder mehrere Markierungen auf der Membran vorgenommen werden. An diesen Markierungen können dann ein oder mehrere Löcher in die Membran eingebracht werden, beispielsweise mit einem Lochwerkzeug, wie etwa einer Kofferdam-Lochzange, welches ebenfalls Bestandteil des Operations-Sets sein kann, aber nicht sein muss.

Eine oben genannte Benutzungsanleitung enthält Anweisungen an den Benutzer, beispielweise den Zahnarzt, die ihn anweisen, in die Membran ein oder mehrere Löcher derart einzubringen, dass die Zahnwurzel mindestens eines Zahnes oder der Implantatkörper mindestens eines Zahmimplantates, bevorzugt die Zahnwurzeln mehrerer benachbarter Zähne oder die Implantatkörper mehrerer benachbarter Zahnimplantate, von dem Loch bzw. den Löchern insbesondere im Wesentlichen manschettenartig umschliessbar ist. Optional kann die Benutzungsanleitung auch Anweisungen oder Empfehlungen enthalten, die dem Benutzer erläutern, an welcher/n Position(en) und/oder auf welche Weise das mindestens eine Loch in die Membran einzubringen ist. Eine solche Benutzungsanleitung kann beispielsweise auf Papier gedruckt und dem Operations-Set beigefügt sein.

Abgesehen von den oben beschriebenen Verwendungen kann die erfindungsgemässe Membran auch für viele weitere Indikationen im Bereich der Zahnmedizin oder der Orthopädie angewendet werden, wie beispielsweise für die Behandlung von Osteoporose oder Knochenbrüchen.

Im Folgenden wird die Erfindung anhand von Ausführungsbeispielen und Figuren näher erläutert. Dabei zeigen
- Figur 1:: einen Furkationsdefekt vor der Behandlung;
- Figur 2:: den Furkationsdefekt gemäss Figur 1 mit einer nicht erfindungsgemässen Membran aus Teflon;
- Figur 3:: eine nicht erfindungsgemässe Membran, die auf einen Zahn gelegt und mit einem Zahnumschlingungsdraht fixiert wurde;
- Figur 4:: eine nicht erfindungsgemässe Membran, die frei an einem Zahn platziert wurde;
- Figur 5:: eine erste erfindungsgemässe Membran mit drei Löchern;
- Figur 6:: die auf ein Modell eines Unterkiefers applizierte erfindungsgemässe Membran gemäss Figur 6, zirkulär die Zahnwurzeln 31-41-42 umfassend, bukkal und lingual überlappend;
- Figur 7:: das Modell gemäss Figur 6, jedoch mit reponiertem Zahnfleisch;
- Figuren 8a bis c:: drei Ansichten einer zweiten erfindungsgemässen Membran mit einem Loch und zwei Schichten.

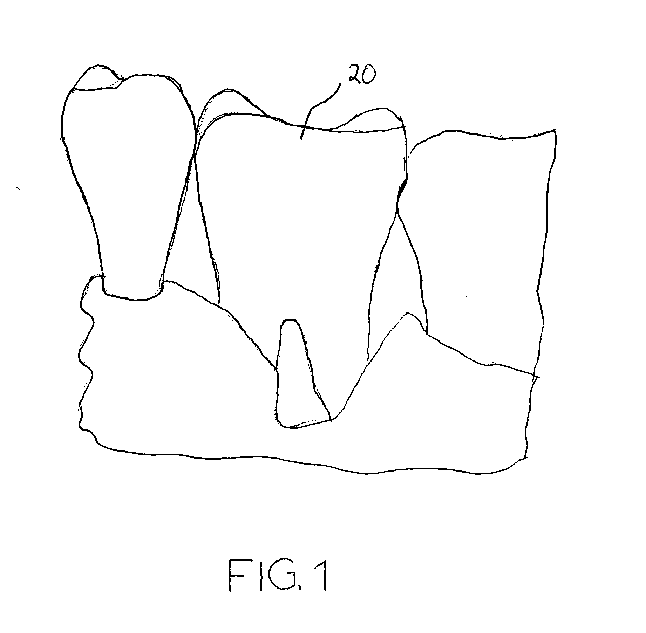

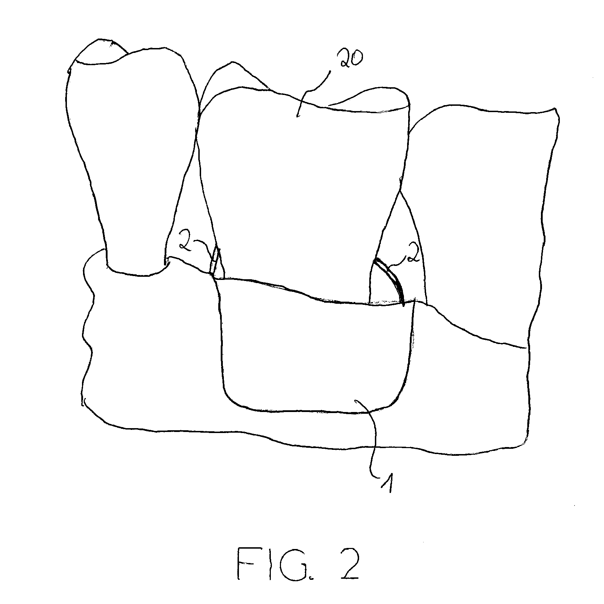

In Figur 1 ist ein Furkationsdefekt unterhalb eines mehrwurzligen Zahns 20 dargestellt. In Figur 2 ist der gleiche Furkationsdefekt mit einer Membran 1 aus Teflon gezeigt, die nicht resorbier und daher nicht erfindungsgemäss ist. Die Membran 1 musste mit einer Zahnumschlingungsnaht 2 gedeckt werden, um sie zu fixieren. Aufgrund der fehlenden Resorbierbarkeit der Membran 1 muss diese in einem zweiten chirurgischen Eingriff wieder entfernt werden, was sehr kostenintensiv ist und zudem für den Patienten eine zusätzliche Belastung darstellt.

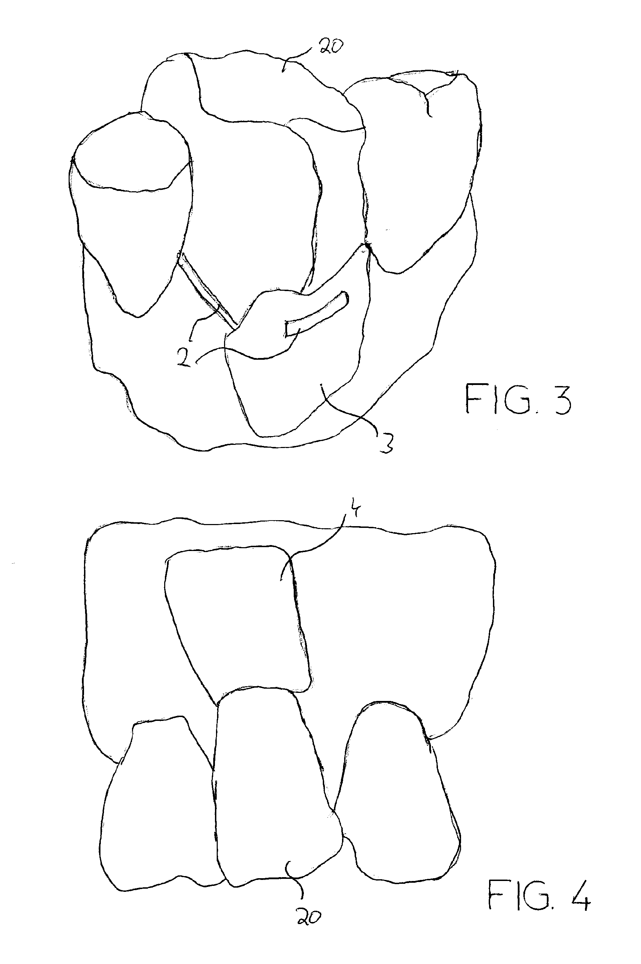

In Figur 3 ist ein Zahn 20 mit einer ebenfalls nicht erfindungsgemässen Membran 3 zu erkennen, die mit einer Zahnumschlingungsnaht 2 fixiert werden musste.

Die in Figur 4 dargestellte, ebenfalls nicht erfindungsgemässe Membran 4 ist frei platziert. Auch diese Membran 4 sollte jedoch zusätzlich fixiert werden, um einen sichereren Halt zu gewährleisten.

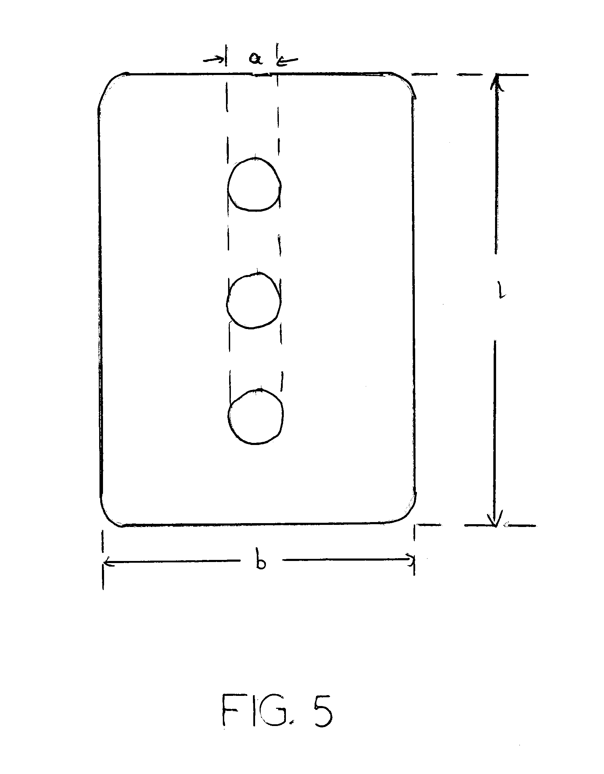

Die Figur 5 zeigt eine erfindungsgemässe Membran 10. Diese enthält drei nebeneinander angeordnete Löcher 11 mit einem Durchmesser a von 3 mm, die derart ausgebildet sind, dass die Zahnwurzeln mehrerer benachbarter Zähne von jeweils einem der Löcher 11 im Wesentlichen manschettenartig umschliessbar sind. Die Membran 10 hat eine Länge l von 25 mm und eine Breite b von 17 mm.

Die Membran 10 besteht aus resorbierbarem Polyurethan und kann beispielsweise nach einem der im Folgenden beschriebenen Rezepturen und Verfahren hergestellt werden kann:

### Beispiele A bis C:

### Rezeptur:

| **Rohstoff** | **Erläuterung/ Bezugsquelle** | **Bsp. A Menge** **[g]** | **Bsp. B Menge** **[g]** | **Bsp. C Menge** **[g]** |
|---|---|---|---|---|
| PEG-400 | Polyethylenglycol, Molekülmasse 400, erhältlich von Kolb AG, Hedingen, Schweiz | 8 | 8 | 8 |
| PEG-4000 | Polyethylenglycol, Molekülmasse 4000 | 6 | 0 | 12 |
| Sympatens TRH/400 | erhältlich von Kolb AG, Hedingen, Schweiz | 0, 02 | 0, 02 | 0, 02 |
| Capa™ 4801 | Tetrafunktionales Polycaprolacton mit durchschnittlicher Molmasse von 8000, erhältlich von Perstorp UK Ltd., Warrington, UK | 6 | 12 | 0 |
| Rizinusöl | Ph. Eur., Alberdingk Boley, Krefeld, Deutschland | 4 | 4 | 4 |
| Coscat® 83 | Urethan-Katalysator, erhältlich von Vertellus Inc., Indianapolis, USA | 0,14 | 0,14 | 0,14 |
| Luvitec® K30 | Polyvinylpyrrolidon, erhältlich von BASF, Deutschland | 0, 84 | 0, 84 | 0, 84 |
| Calciumstearat | | 0,5 | 0,5 | 0,5 |
| Türkischrotöl | sulfoniertes Rizinusöl | 0 | 0 | 0,1 |
| Desmodur® E 305 | NCO-Präpolymer auf Basis Hexamethylendiisocyanat, erhältlich von Bayer Material Science AG, Leverkusen, Deutschland | 16,3 | 16,3 | 16,3 |

Zunächst wurde Capa™ 4801 geschmolzen. Anschliessend wurden PEG-400 und Luvitec® K30 hinzugefügt, und die Mischung wurde in einem hitzebeständigen Gefäss mit Hilfe eines Spatels bei einer Temperatur von 170 °C gemischt, bis sich eine klare Lösung ergab. Rizinusöl und Sympatens TRH/400 als Emulgator wurden daraufhin hinzugefügt, und die Mischung wurde mit Hilfe eines Spatels gründlich durchgemischt. Anschliessend wurde die Mischung auf eine Temperatur von 70 °C gebracht und in einen PP-200-Becher (geeignet für das Speedmixer™-System) überführt, woraufhin Coscat® 83 und Calciumstearat hinzugefügt wurden. Auf 70 °C vorgeheiztes Desmodur® E 305 wurde hinzugegeben. Es wurde dann mittels eines Speedmixer™ 400 FVZ (Hauschild & Co KG, Hamm, Deutschland) während einer Minute bei 2700 U/min vermischt. Das resultierende Gemisch wurde umgehend mit Hilfe einer Rakel auf silikonisiertem Papier zu Membranen mit Dicken von 100 µm und 300 µm ausgerakelt. Das silikonisierte Papier wurde anschliessend entfernt. Schliesslich wurden die Löcher 11 mit Hilfe einer Kofferdam-Lochzange eingebracht.

### Beispiel D:

### Rezeptur:

| **Rohstoff** | **Erläuterung/ Bezugsquelle** | **Menge [g]** |
|---|---|---|
| PEG-400 | Polyethylenglycol, Molekülmasse 400, erhältlich von Kolb AG, Hedingen, Schweiz | 9,2 |
| Kollidon® 17 PF | Polyvinylpyrrolidon, erhältlich von BASF, Deutschland | 1,06 |
| PEG-4000 | Polyethylenglycol, Molekülmasse 4000, erhältlich von Kolb AG, Hedingen, Schweiz | 12,0 |
| | | |
| Coscat® 83 | Urethan-Katalysator, erhältlich von Vertellus Inc., Indianapolis, USA | 0,28 |
| Jeffcat® DMDEE | 2,2-Dimorpholinodiethylether, erhältlich von Alfa Chemicals Ltd., Berkshire, UK | 0,23 |
| Calciumstearat | | 0,5 |
| Türkischrotöl | sulfoniertes Rizinusöl | 0,2 |
| Wasser | | 0,4 |
| Desmodur® E 305 | NCO-Präpolymer auf Basis Hexamethylendiisocyanat, , erhältlich von Bayer Material Science AG, Leverkusen, Deutschland | 24,75 |

Zunächst wurde ein Gemisch aus PEG-4000, PEG-400 und Kollidon® 17 PF in einem hitzebeständigen Gefäss auf einer Heizplatte aufgeschmolzen, so dass ein klares Gemisch entstand. Nach Abkühlen auf 70 °C wurden Coscat® 83, Jeffcat® DMDEE, Calciumstearat, Türkischrotöl und Wasser eindispergiert. In einem PP-200-Becher (geeignet für das Speedmixer™-System) wurde diesem Gemisch nun Desmodur® E 305, das im Ofen auf 70 °C vorgewärmt worden war, zugegeben. Es wurde mittels eines Speedmixer™ 400 FVZ (Hauschild & Co KG, Hamm, Deutschland) während einer Minute bei 2700 U/min vermischt. Das resultierende Gemisch wurde umgehend mit Hilfe einer Rakel auf silikonisiertem Papier zu Membranen mit Dicken von 100 µm und 300 µm ausgerakelt. Das silikonisierte Papier wurde anschliessend entfernt. Schliesslich wurden die Löcher 11 mit Hilfe einer Kofferdam-Lochzange eingebracht.

Die Membran 10 enthält Poren, die allesamt Porendurchmesser von weniger 5 µm haben. Sie ist wasserdampfdurchlässig, wasserdurchlässig, transparent und zelldicht. Optional kann die Membran 10 zusätzlich zu den oben genannten Inhaltsstoffen mindestens ein Biozid (wie etwa Chlorhexidin) und/oder mindestens einen Wachstumsfaktor (wie etwa FGF) enthalten.

Die nachfolgende Tabelle 1 zeigt Wasserdampfdurchlässigkeiten (Feuchtigkeitstransportraten bei Kontakt mit Wasserdampf) der oben beschriebenen Membranen, die gemäss den Abschnitten 3.1 und 3.2 der Norm DIN EN 13726-2:2002 bestimmt wurden, wobei als Prüfdauer gemäss Abschnitt 3.2.3.6 dieser Norm eine Zeit von 24 h gewählt wurde.

**Tabelle 1: Wasserdampfdurchlässigkeiten**

| **Beispiel** | **Dicke der Membran** **[µm]** | **Wasserdampfdurchlässigkeit** **[g/ (m²·24 h) ]** |
|---|---|---|
| Beispiel A | 100 | 2064 |
| Beispiel A | 300 | 1694 |
| Beispiel B | 100 | 1194 |
| Beispiel B | 300 | 472 |
| Beispiel D | 1000 | 1473,3 |
| Beispiel D | 2000 | 1490,0 |

In der nachfolgenden Tabelle 2 sind Wasserdurchlässigkeiten (Feuchtigkeitstransportraten bei Kontakt mit Flüssigkeit) der oben beschriebenen Membranen wiedergegeben, die gemäss den Abschnitten 3.1 und 3.3 der Norm DIN EN 13726-2:2002 bestimmt wurden, wobei als Prüfdauer gemäss Abschnitt 3.3.3.3 dieser Norm eine Zeit von 4 h gewählt wurden.

**Tabelle 2: Wasserdurchlässigkeiten**

| **Beispiel** | **Dicke der Membran [µm]** | **Wasserdurchlässigkeit** **[g/(m²·24 h)]** |
|---|---|---|
| Beispiel A | 100 | 24708 |
| Beispiel A | 300 | 19524 |
| Beispiel B | 100 | 1884 |
| Beispiel B | 300 | 780 |
| Beispiel D | 1000 | 4740,0 |
| Beispiel D | 2000 | 2560,0 |

Des Weiteren wurden auch die Zugfestigkeiten der Membranen bestimmt, und zwar sowohl im trockenen als auch im nassen Zustand. Bei den Versuchen mit einer Zugfestigkeits-Testvorrichtung von Instron wurde eine kleine Probe der Membran mit einer Fläche von 20 cm² mit einer kontrollierten, graduell anwachsenden Kraft gestreckt, bis sich die Form der Probe änderte oder die Probe riss. Der nasse Zustand wurde erhalten, indem die Probe für 15 min in destilliertem Wasser eingeweicht wurde.

**Table 3: Zugfestigkeiten im trockenen Zustand**

| **Beispiel** | **Bruchspannung [MPa]** | **Dehnung beim Bruch [%]** | **Spannung bei Dehnung von 20 % [MPa]** | **Spannung bei Dehnung von 40 % [MPa]** | **Spannung bei Dehnung von 60 % [MPa]** | **Spannung bei Dehnung von 100 % [MPa]** |
|---|---|---|---|---|---|---|
| A (100 µm) | 3,298 | 870,4 | 0,858 | 1,050 | 1,135 | 1,235 |
| A (300 µm) | 3,126 | 1074,1 | 0,541 | 0,684 | 0,746 | 0,813 |
| B (100 µm) | 7,714 | 708,8 | 2,185 | 2,518 | 2,683 | 2,897 |
| B (300 µm) | 6,746 | 727,5 | 2,385 | 2,740 | 2,898 | 3,062 |

**Table 4: Zugfestigkeiten im nassen Zustand**

| **Beispiel** | **Bruchspannung [MPa]** | **Dehnung beim Bruch [%]** | **Spannung bei Dehnung von 20 % [MPa]** | **Spannung bei Dehnung von 40 % [MPa]** | **Spannung bei Dehnung von 60 % [MPa]** | **Spannung bei Dehnung von 100** % **[MPa]** |
|---|---|---|---|---|---|---|
| A (100 µm) | 0,038 | 243,1 | 0,085 | 0,111 | 0,129 | 0,148 |
| A (300 µm) | 0,026 | 150,8 | 0,056 | 0,059 | 0,058 | 0,049 |
| B (100 µm) | 2,810 | 312,5 | 1,517 | 1,872 | 2,063 | 2,290 |

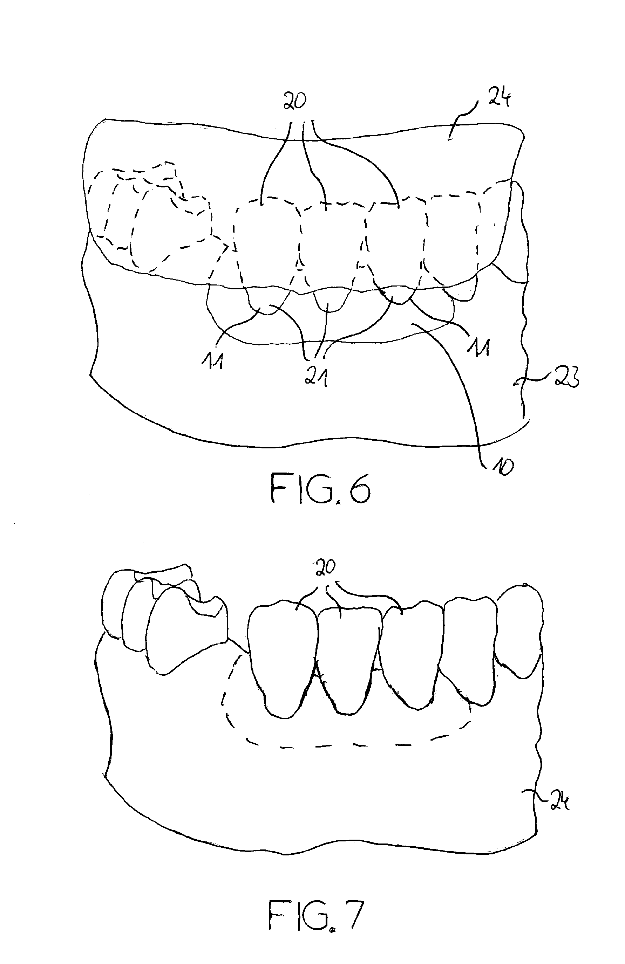

Die Figur 6 lässt ein Modell eines Unterkiefers 23 mit mehreren Zähnen 20 und aufgeklapptem Zahnfleisch 24 erkennen. Auf dem Unterkiefer 23 ist eine erfindungsgemässe Membran 10 angeordnet. Diese Membran 10 weist drei nebeneinander angeordnete Löcher 11 mit einem Durchmesser von jeweils 3 mm auf. Diese Löcher 11 können beispielsweise mit Hilfe einer Kofferdam-Lochzange eingebracht worden sein. Die Zahnwurzeln 21 von drei benachbarten Zähnen 20 sind von jeweils einem der Löcher 11 manschettenartig umschlossen. Die Elastizität und Flexibilität der Membran 10 ermöglicht den anhaltend dichten und vertikal stabilen Defekt-Verschluss im kritischen cervicalen Bereich zirkulär um die Zahnwurzeln 21 der benachbarten Zähne 20, ohne dass die Membran Kräfte zwischen den Zähnen aufbaut.

Figur 7 zeigt die Situation aus Figur 6 mit reponiertem Zahnfleisch 24. Direkt unterhalb des Zahnfleisches 24 kann die Membran 10 temporär die Funktion des Saumepithels ersetzen und gleichzeitig und ohne weitere Fixierung der Separation von Weichgewebe und Defektvolumen dienen. Ein Zweiteingriff erübrigt sich bei komplikationsloser Heilung infolge der Resorption.

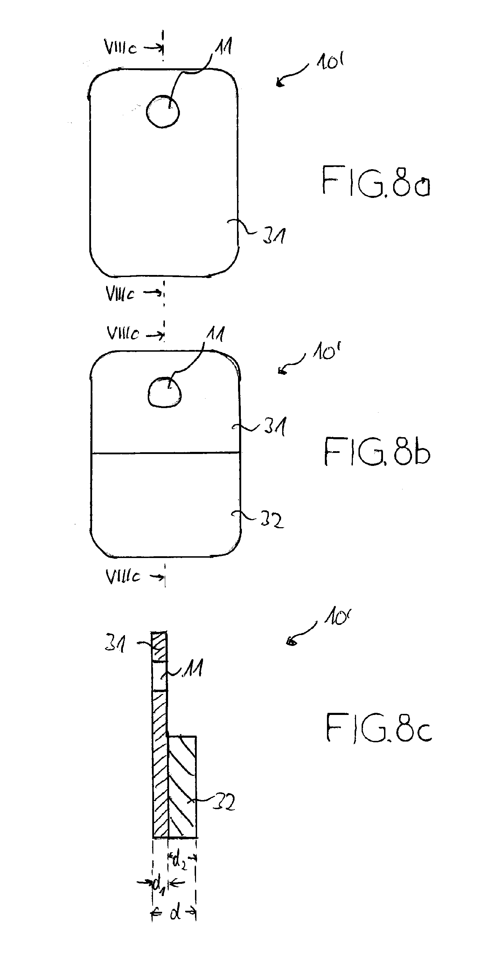

Eine zweite erfindungsgemässe Membran 10' ist in den Figuren 8a bis 8c dargestellt, die eine erste Schicht 31 und eine zweite Schicht 32 enthält. Figur 8a zeigt eine Draufsicht auf eine erste Seite der Membran 10', in der nur die erste Schicht 31 erkennbar ist. Gemäss der Draufsicht auf eine zweite gegenüberliegende Seite der Membran 10' bedeckt die zweite Schicht 32 die erste Schicht 31 nur in einem Deckungsbereich, der nur einen Teilbereich der ersten Schicht 31 bildet. Ein Loch 11 der Membran 10' ist ausserhalb des Deckungsbereichs angeordnet; es durchdringt also nur die erste Schicht 31, nicht aber auch die zweite Schicht 32. Selbstverständlich ist es im Rahmen der Erfindung aber ebenfalls möglich, dass das Loch / die Löcher im Bereich der Doppelschicht liegen und somit beide Schichten durchmessen.

Figur 8c zeigt eine seitliche Schnittansicht entlang der Schnittlinie VIIIc. Die erste Schicht 31 hat eine Dicke d₁ von 0,5 mm und eine maximale Porengrösse von 5 µm, und die zweite Schicht 32 hat eine Dicke d₂ von 1,5 mm und eine Porosität von 70% bei einer maximalen Porengrösse von 0,25 mm. Im Deckungsbereich hat die Membran 10' also eine Gesamtdicke d = d₁ + d2 = 2,0 mm.

Die spezielle Ausbildung der Membran 10' gemäss Figuren 8a bis 8c ermöglicht es, dass nur die erste Schicht 31 eine hier nicht dargestellte Zahnwurzel oder einen hier nicht dargestellten Implantatkörper manschettenartig umschliessen kann. Die Membran 10' kann dabei mit der ersten, in Figur 8a erkennbaren Seite in Richtung auf das Hartgewebe platziert werden. Die gegenüberliegende zweite, in Figur 8b sichtbare Seite ist dann in Richtung auf das Weichgewebe ausgerichtet und dann das Wachstum dieses Weichgewebes erleichtern.

## Patentansprüche

1. Membran (10; 10'), enthaltend mindestens ein Loch (11), welches derart ausgebildet ist, dass die Zahnwurzel (21) mindestens eines Zahnes (20) oder der Implantatkörper mindestens eines Zahnimplantates, bevorzugt die Zahnwurzeln (21) mehrerer benachbarter Zähne (20) oder die Implantatkörper mehrerer benachbarter Zahnimplantate, von dem Loch (11) bzw. den Löchern (11) insbesondere im Wesentlichen manschettenartig umschliessbar ist,
**dadurch gekennzeichnet, dass**
die Membran (10; 10') mindestens ein synthetisches und resorbierbares Material enthält oder daraus besteht.

2. Membran (10; 10') gemäss einem der vorangehenden Ansprüche, **dadurch gekennzeichnet, dass**
sie mindestens ein Polymer enthält oder daraus besteht, welches ausgewählt ist aus der Gruppe, die besteht aus: Polyurethan, Poly-α-Hydroxyester, Polyorthoester, Polyetherester, Polyanhydrid, Polyphosphazen, Polypropylenfumarat, Polyesteramid, Polyethylenfumarat, Polyaminosäuren, Polysaccharide, Polypeptide, Polyhydroxybutyrat, Polyhydroxyvalerates, Polymalat, Poly(Milchsäure), Poly(Glykolsäure), Polycaprolacton, Poly(glycolid-co-trimethylencarbonat), Polydioxanon, Polyethylenglycol, sowie Gemische aus diesen Polymeren.

3. Synthetische und resorbierbare Membran (10; 10') gemäss Anspruch 2,
**dadurch gekennzeichnet, dass**
die Membran (10; 10') hergestellt oder herstellbar ist aus einer Zusammensetzung, welche enthält:
a) wenigstens eine Polyol-Komponente enthaltend wenigstens einen Stoff mit wenigstens zwei, bevorzugt genau zwei, genau drei oder genau vier Hydroxyl-Gruppen;
b) wenigstens eine Polyisocyanat-Komponente oder wenigstens ein Polyurethan-Präpolymer enthaltend wenigstens einen Stoff mit wenigstens zwei, bevorzugt genau zwei, genau drei oder genau vier Isocyanat-Gruppen;
c) ein Additiv enthaltend wenigstens einen der folgenden Stoffe: Polyvinylpyrrolidon, Polyvinylpolypyrrolidon, sulfonierte Copolyester, Polyvinylalkylether, ein Copolymer aus Polyvinylpyrrolidon mit Vinylacetat, Vinylcaprolactam oder Vinylimidazol, ein Copolymer aus Alkylvinylether mit einer Säure oder einem Anhydrit, vorzugsweise Maleinsäure oder Maleinanhydrit, sowie Polymere und Copolymere von Acrylsäure und deren Salzen oder Mischungen davon.

4. Membran (10; 10') gemäss einem der vorangehenden Ansprüche, **dadurch gekennzeichnet, dass**
sie eine Dicke (d) im Bereich von 0,01 mm bis 4 mm, bevorzugt von 0,05 mm bis 1,0 mm, besonders bevorzugt von 0,1 mm bis 0,8 mm aufweist.

5. Membran (10; 10') gemäss einem der vorangehenden Ansprüche, **dadurch gekennzeichnet, dass**
sie Poren mit Porendurchmessern im Bereich von 0,0001 mm bis 0,05 mm, bevorzugt von 0,0005 mm bis 0,01 mm, besonders bevorzugt von 0,001 mm bis 0,005 mm aufweist.

6. Membran (10; 10') gemäss einem der vorangehenden Ansprüche, **dadurch gekennzeichnet, dass**
sie gasdurchlässig, insbesondere wasserdampfdurchlässig ist und insbesondere eine Wasserdampfdurchlässigkeit aufweist, die mindestens 100 g/(m²·24 h), bevorzugt mindestens 300 g/(m²·24 h) und besonders bevorzugt mindestens 500 g/ (m²·24 h) bis 30000 g/ (m²·24 h) ist.

7. Membran (10; 10') gemäss einem der vorangehenden Ansprüche, **dadurch gekennzeichnet, dass**
sie eine Wasserdampfdurchlässigkeit aufweist, die höchstens 400000 g/ (m²·24 h), bevorzugt höchstens 200000 g/ (m²·24 h) und besonders bevorzugt höchstens 50000 g/(m²·24 h) ist.

8. Membran (10; 10') gemäss einem der vorangehenden Ansprüche, **dadurch gekennzeichnet, dass**
sie wasserdurchlässig ist und insbesondere eine Wasserdurchlässigkeit von mindestens 100 g/(m²·24 h), bevorzugt mindestens 300 g/(m²·24 h) und besonders bevorzugt mindestens 500 g/(m²·24 h) aufweist.

9. Membran (10; 10') gemäss einem der vorangehenden Ansprüche, **dadurch gekennzeichnet, dass**
sie eine Wasserdurchlässigkeit von höchstens 400000 g/ (m²·24 h), bevorzugt höchstens 200000 g/ (m²·24 h) und besonders bevorzugt höchstens 50000 g/(m²·24 h) aufweist.

10. Membran (10; 10') gemäss einem der vorangehenden Ansprüche, **dadurch gekennzeichnet, dass**
sie transparent ist.

11. Membran (10; 10') gemäss einem der vorangehenden Ansprüche, **dadurch gekennzeichnet, dass**
sie mindestens ein Biozid enthält, insbesondere ausgewählt aus der Gruppe bestehend aus Bakteriziden, Fungiziden, Viruziden, Antibiotika, Antimykotika und beliebigen Kombinationen davon, wie etwa Polyhexanid, Chlorhexidin, Silber oder Taurolidin, Octenidin-Dihydrochlorid, Cetylpyridiniumchloric, Triclosan oder beliebigen Kombinationen davon.

12. Membran (10; 10') gemäss einem der vorangehenden Ansprüche, **dadurch gekennzeichnet, dass**
sie mindestens einen Wachstumsfaktor enthält, insbesondere ausgewählt aus der Gruppe bestehend aus FGF, TGF, PDGF, EGF, VEG, IGF, NGF, Interleukine und beliebigen Kombinationen davon.

13. Membran (10; 10') gemäss einem der Ansprüche 11 und 12,
**dadurch gekennzeichnet, dass**
das Biozid und/oder der Wachstumsfaktor im Bereich mindestens einer Oberfläche der Membran (10; 10') vorhanden ist.

14. Membran (10; 10') gemäss einem der Ansprüche 11 bis 13,
**dadurch gekennzeichnet, dass**
das Biozid und/oder der Wachstumsfaktor im Inneren der Membran (10; 10') vorhanden ist.

15. Operations-Set enthaltend mindestens eine Membran (10; 10'), welche mindestens ein synthetisches und resorbierbares Material enthält oder daraus besteht, insbesondere mindestens eine Membran (10; 10') gemäss einem der vorangehenden Ansprüche, sowie
- mindestens ein Lochwerkzeug zum Einbringen mindestens eines Lochs (11) in die Membran (10; 10'), insbesondere mindestens eine Kofferdam-Lochzange, und/oder
- mindestens eine Spannzange zum Spannen der Membran (10; 10') und/oder
- mindestens ein Spreizwerkzeug zur kurzzeitigen Erweiterung eines Interdentalraumes zum erleichterten Applizieren der Membran (10; 10') und/oder
- mindestens eine Schablone zur Vorgabe der relativen Positionen mehrerer in die Membran (10; 10') einzubringender Löcher (11) und/oder
- mindestens einen Stempel zum Markieren der Position mindestens eines in die Membran (10; 10') einzubringenden Lochs (11) und/oder
- mindestens eine Benutzungsanleitung zum Einbringen mindestens eines Lochs (11) in die Membran (10; 10'), insbesondere mit Hilfe mindestens eines Lochwerkzeugs, insbesondere einer Kofferdam-Lochzange.
